(19) Europäisches Patentamt
European Patent Office
Office européen des brevets

(11) Publication number: 0 184 672 B1

(12) EUROPEAN PATENT SPECIFICATION

(45) Date of publication of patent specification: 30.08.89

(51) Int. Cl.[4]: G 01 N 33/66, G 01 N 33/52

(21) Application number: 85114164.8

(22) Date of filing: 07.11.85

# (54) Nonenzymatic glucose test.

(30) Priority: 19.11.84 US 673184
20.05.85 US 736300

(43) Date of publication of application:
18.06.86 Bulletin 86/25

(45) Publication of the grant of the patent:
30.08.89 Bulletin 89/35

(84) Designated Contracting States:
AT BE CH DE FR GB IT LI LU NL SE

(56) References cited:
WO-A-82/01804

CHEMICAL ABSTRACTS, vol. 79, no. 13, 1st October 1973, page 527, no. 79070k, Columbus, Ohio, US; S.P. MOULIK et al.: "Fundamental studies on interaction of carbohydrates with alkaline-earth metals. III. Reaction of D-glucose and maltose with the hydroxides of barium, calcium, magnesium, and strontium cations in presence of ethylenediamine and 2-amino-ethanol"

(73) Proprietor: MILES INC.
1127 Myrtle Street
Elkhart Indiana 46514 (US)

(72) Inventor: Greene, Carmine
52996 Highland Drive
South Bend IN 46635 (US)
Inventor: Ismail, Ibrahim A.
1144 Manchester Drive
South Bend IN 46615 (US)
Inventor: Wu, Wen H.
51701 Northfield Drive
Elkhart, IN 46514 (US)

(74) Representative: Dänner, Klaus, Dr. et al
c/o Bayer AG Konzernverwaltung RP Patente Konzern
D-5090 Leverkusen 1 Bayerwerk (DE)

EP 0 184 672 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to a nonensymatic, semiquantitative determination of glucose in general and to a nonenzymatic diagnostic composition for the colorimetric determination of glucose in aqueous test samples in particular.

Determination of glucose concentration in aqueous solution is useful industrially in the sugar industry and medically. Medically, the semiquantitative determination of glucose in body fluids, such as urine or blood, is of importance as a public health measure to screen large numbers of people for diabetes, and is of particular importance for diabetic patients who must control their sugar intake. Because early diagnosis and continued control are so important in diabetes, a glucose test, to be of greatest value to the physician, clinician or home diabetic user must be rapid and single enough to perform conveniently and yet sensitive enough to reflect meaningful variations in urine or blood glucose.

Semiquantitative determination of high range glucose, defined herein as glucose concentrations of 1,000 milligrams per deciliter (mg/dl) and above, is important because urine glucose concentration in diabetic patients can range up to 5,000 mg/dl or higher. The quantitative estimation of high urine glucose concentrations is important for at least two reasons. First, in emergency situations it is important to determine whether a state of unconsciousness can be attributed to diabetic coma, which would be indicated by a high urine glucose concentration. Second, urine glucose levels become elevated if an insufficient amount of insulin has been administered. A test which can estimate high urine glucose concentrations therefore has utility in the therapeutic monitoring of insulin requirements.

Most diagnostic testing for glucose presently performed clinically is based on the enzymatic action of glucose oxidase on β-D-glucose:

$$\beta\text{-D-glucose} \xrightarrow{\text{[glucose oxidase]}} H_2O_2 + \text{gluconic acid} + O_2 + H_2O$$

and the resultant oxidation of a chromogen (Cr) to its oxidized state (Cr*) which is visually detectable by a color change:

$$H_2O_2 + Cr \xrightarrow{\text{[Peroxidase]}} H_2O + Cr^*$$

Great convenience is obtained when the test device can be used semiquantitatively to determine glucose levels by visual comparison of the color, developed after contact with a test sample, with an appropriate color chart. Such semiquantitative determinations can also be performed instrumentally by measuring the refectance of a reacted device. However, as the concentration of glucose increases above 1,000 mg/dl the color of most chromogens used in enzymatic systems is so dark as to preclude distinguishing high concentration levels. US—A—4,340,669 describes the observed results with o-tolidine, tetramethylbenzidine and tetraethylbenzidine as chromogen at 0, 50, 100, 250, 500 and 1,000 mg/dl glucose in the fluid being tested. Each of these chromogens turns from yellow to bright green when the concentration of glucose increases from 0 to 50 mg/dl. As the concentration of glucose increases above 500 mg/dl the color of the oxidized chromogen darkens so that the observed colors of the respective chromogens were olive-black, black and deep green. This observation highlights a problem with the semiquantitative enzymatic determination of glucose in aqueous fluids, that is at high concentrations known chromogens appear black or very dark green thereby limiting the utility of the test devices for determination of glucose above 500 mg/dl. While the problem is not so acute if the color change is determined instrumentally, it nonetheless still exists. Some success in incresaing the visually readable range of glucose with enzymatic compositions has been achieved by the addition of secondary chromogens such as m-anisidine (US—A—4,340,669).

In addition to poor color differentiation at high glucose concentrations, enzyme-based glucose tests are interfered with by ascorbic acid (vitamin C) present in body fluids, are expensive and are subject to stability problems.

Nonenzymatic methods for measuring glucose have also been used. These include instrumental methods based on a measuring electrode (see, for example, US—A—4,127,448) and even a non-invasive automatic glucose sensor system which scans the patient's eye for radiation transmitted through the cornea (see US—A—3,958,560). US—A—4,278,438 discloses a method and apparatus for the analysis of saccharides. An alkylene polyamine prepared in a borate buffer is used to elute saccharides from a chromatography column.

US—A—4,371,374 discloses a method of monitoring blood glucose by separating and quantitating nonenzymatic glycosylated amino acids, peptides or mixtures thereof by treating a urine sample with a suitable boronic acid to complex the glycosylated compounds, separating them and analyzing the separated complexed material.

The present invention does not require elaborate equipment, but nevertheless allows the determination of glucose up to any desired concentration level, by use of the complexation of glucose with a borate buffer. Complexation of sugars with boron and alkali earth metal dihydroxides has been reported

[S. A. Barker et al., *Carbohydrate Research*, 26 (1973) 33—40; N. Roy *et al.*, *Carbohydrate Research*, 24 (1972) 180—183]; but this phenomenon has not been used to solve the problem of semiquantitatively determining glucose concentration in an aqueous test samples.

A preferred embodiment of the present invention provides a self-indicating device for the determination of glucose based on the use of the complexation of glucose with a borate buffer. The self-indicating device of this invention permits a visual determination of the concentration of glucose without comparison to a color indicator chart.

A disposable indicator device for the determination of cholesterol was disclosed in US—A—4,042,329. The disclosed device provides an indication of the concentration of cholesterol in a given biological fluid which is directly readable in notation format.

The drawing indicates the reproducibility of visual determinations of glucose concentration with a nonenzymatic test device of the present invention. Test devices formulated for the semiquantitative determination of high range glucose were contacted with contrived urine test samples containing from 1 g/dl to 8 g/dl glucose. The graph verifies the linear relationship of glucose concentration, G. in g/dl with device reading, R, in g/dl. The test devices were prepared by pretreating a paper carrier matrix with a borate buffer prepared from phenylboronic acid prior to incorporation of a test composition comprising a borate buffer prepared from boric acid and a pH indicator.

The invention provides a test composition for semi-quantitatively determining at least 1,000 mg/dl glucose in an aqueous test sample, a test device and describes a method for its preparation and use.

The test composition comprises: a) a borate buffer capable of moderating pH change in a pH range of from pH 6.5 to pH 12; and b) a pH indicator capable of providing a detectable colorimetric response in a pH range of from pH 6.5 to pH 12. A carrier matrix can be incorporated with the test composition to provide a particularly convenient test device format.

A number of carbohydrates containing a cisdiol grouping from a variety of complexes with compounds containing a dihydroxide group. (See, for example, N. Roy *et al., Carbohydrate Research* 24 (1972) 180—183, and S. A. Barker *et al., Carbohydrate Research*, 26 (1973) 33—40). It has been found that this complex formation can be used to provide a semiquantitative determination of the concentration of glucose in an aqueous test sample by preparing a test solution by contacting the sample with a borate buffer, at an initial pH above 6.5, capable of forming a complex with glucose, which complex formation releases a proton into solution, and determining the final pH of the test solution.

The borate buffer comprises boron dihydroxides of the general formula:

$$Z—B(OH)_2$$

wherein Z is an electron withdrawing group such as a nitro group or electron stabilizing group such as a hydroxyl or arene group. Where Z is a hydroxyl group the boron dihydroxide is boric acid. Suitable boron dihydroxides include boric acid, phenylboronic acid, p-nitrophenylboronic acid, 4-methoxyphenylboronic acid and α-naphthylboronic acid, naphthylboronic acid as well as other areneboronic acids and their derivatives. An arene groups is defined as any hydrocarbon group containing at least one aromatic ring. These groups are useful in the present invention provided the anionic negative form of the dihydroxide can be stabilized by electron resonance over the aromatic ring. For example, the compound phenylboronic acid where Z is a phenyl group is particularly useful in the invention. In addition arene derivatives, such as p-nitrophenylboronic acid, which contain electron withdrawing groups as substituents on the aromatic ring are also useful.

Glucose can be determined by preparing a test solution with an aqueous test sample and a borate buffer at an initial pH above 6.5, capable of forming a complex with glucose, which complex formation releases a proton, and measuring the final pH of the test solution. The test solution can usually be formed by simply contacting the dihydroxide with the aqueous test sample.

The boron dihydroxides generally form 1:1 complexes with glucose. Therefore the ratio of dihydroxide to glucose in the test sample must be approximately one to one. To form a test solution of a boron dihydroxide, such as boric acid, in sufficient concentration to determine glucose concentrations of about 500 mg/dl or greater, it may be necessary to use a base such as potassium hydroxide or sodium hydroxide to dissolve the boron dihydroxide. An equivalent procedure would be the use of the salt form of boric acid as part of the hydroxide component. Obviously, other bases are also useful provided they do not interfere with the complex formation between the dihydroxide and glucose.

The final pH of the test solution can be measured visually or instrumentally after the addition of a pH indicator.

The pH change which occurs with complex formation between the dihydroxide component and glucose, can be moderated by the addition of a buffer. A test composition which includes a buffer capable of moderating a pH change over the pH range of from pH 6.5 to pH 12 can be used to determine glucose over a wider concentration range than a test composition without such a buffer. Suitable buffers include tris(hydroxymethyl)aminomethane, commonly known as TRIS, N,N-bis(2-hydroxyethylglycine, commonly known as BICINE and N-2-hydroxyethylpiperazine-N′-2-ethanesulfonic acid, commonly known as HEPES. It is particularly convenient when using a boron dihydroxide such as boric acid or phenylboronic acid to use the buffer form as the dihydroxide component.

A borate buffer is defined as the mixture of the acid and base form of a $Z{-}B(OH)_2$ compound. The equilibrium between the two forms can be shown schematically as:

$$Z{-}B(OH)_2 + H_2O \rightleftharpoons Z{-}\overset{\ominus}{B}(OH)_3 + H^+$$

where Z can be any of the groups described previously.

The borate buffer can be prepared from boric acid (Z=OH) or from areneboronic acid derivatives such as phenylboronic acid or mixtures of $Z{-}B(OH)_2$ compounds by commonly used laboratory methods well known to those skilled in the art. For example, a solution of boric acid buffer can be prepared by titrating boric acid with a base such as sodium or potassium hydroxide to an initial pH within the buffering range of the $Z{-}B(OH)_2$ compound used. This buffering range is expected to fall between pH 6.5 to pH 12 for most $Z{-}B(OH)_2$ compounds. The buffer can also be prepared by adding equimolar parts of the acid and base forms of the dihydroxide and dissolving in water.

The choice of the initial buffer pH can affect the concentration range of glucose which can be determined with a particular test composition. For example, the effectiveness of the complexation of boron dihydroxide with glucose decreases as the pH of the solution falls below the pKa of the borate buffer. The addition of electron withdrawing substituents on a buffer prepared from an areneboronic acid derivative changes the pKa of the buffer and therefore its effective pH and complexing range. For example, boric acid, pKa 9.2, has a lower complexing capacity below about pH 7.0, while phenylboronic acid, pKa 8.8, will complex with glucose at a pH as low as 6.5, p-Nitrophenylboronic acid, pKa 7.4, has an even lower effective pH. A mixture of borate buffers, such as boric acid buffer and phenylboronic acid buffer, can extend the effective pH range of the borate glucose complexing and therefore the glucose concentration range which can be determined. When a boric acid buffer is used, an initial pH above 8.0 is preferred. For high range glucose determinations with a boric acid buffer, an initial pH above 9.0 is particularly preferred.

The buffer is most useful in a glucose determination when the initial pH is slightly above its pKa. The buffer can, of course, be supplied in a dry state by removal of water after the initial pH is set. The buffer is capable of providing that pH when reconstituted.

It is particularly convenient to provide a test composition for the determination of glucose including a dihydroxide as defined herein and a pH indicator capable of providing a detectable colorimetric response in the pH range of from pH 6.5 to pH 12. Any pH indicator which changes color within this pH range, or any combination of indicators, can be used. Useful indicators include m-cresol purple, cresol red, neutral red, thymol blue, phenophthalein, o-cresol phthalene, phenol red, bromothymol blue or Universal Indicator, a mixture of indicators available from Kodak. The test composition can be used to determine glucose concentration simply by contacting the composition and the aqueous test sample and observing the detectable colorimetric response produced.

A pH indicator changes color over a range of pH values. The pKa of an indicator represents approximately the midpoint of its color changes. The initial pH of the borate buffer chosen is related to which indicator is used. For example, m-cresol purple changes from purple at pH 9.0 to yellow at pH 7.4. At pH values above 9.0, the indicator remains purple or changes color very little with pH change. If an initial pH of 9.0 is used, a detectable response will be apparent with any pH change. If an initial pH of 9.2 or higher is used, the composition will not change color i.e., will not have a detectable response to glucose concentration, until the pH drops below pH 9.0. Therefore, the compositions utilizing m-cresol purple containing a buffer capable of providing an initial pH of 9.0 will be more sensitive to a lower glucose concentration than such compositions containing a buffer capable of providing an initial pH of 9.2. If a different pH indicator is used, another initial pH can be preferred. For example cresol red has a pH range between about 8.8 (red) and about 7.2 (yellow); a lower initial pH, such as pH 9.0, is then preferred for optimal performance in this test composition. The use of more than one indicator can provide a color change over a broader pH range and therefore over a broader glucose concentration range.

A test composition which is particularly suited for the determination of high range glucose (defined herein as at least 1,000 milligrams glucose per deciliter) is a borate buffer capable of moderating pH change in a pH range of from pH 6.5 to pH 12 and a pH indicator capable of providing a detectable colorimetric response in a pH range of from pH 6.5 to pH 12.

In a preferred composition prepared for high range urine glucose determinations, the borate buffer is a boric acid buffer prepared so that it is capable of providing an initial pH to about 9.2. This high initial pH avoids nonspecific pH change and interference by urine pH and buffering capacity. The buffer so prepared can be provided in a dry state by lyophilization or simply dried to remove the water used to prepare the buffer.

Additional components such as wetting agents, stabilizers or thickeners can be added to the test composition provided they do not interfere with the complexation of the dihydroxide with glucose.

Any of these compositions can be provided in the form of a bottled reagent, a frangible capsule containing the test composition in reagent form, a pill or a tablet.

The test device, a preferred form of the invention, is prepared by treating a suitable carrier matrix with the test composition in the form of a liquid reagent and drying.

The carrier matrix can be any substance capable of being incorporated with the components of the test composition, as long as it is substantially inert with respect to the test composition, porous and/or absorbent relative to the aqueous sample to be tested. The expression "carrier matrix" refers to either bibulous or nonbibulous matrices which are insoluble in and maintain their structural integrity when exposed to water or to other physiological fluids. Suitable bibulous matrices which can be used include paper, cellulose, wood, synthetic resin fleeces, woven and nonwoven fabrics and the like. Nonbibulous matrices include glass fiber, polymer films and microporous membranes.

It is, therefore, to be appreciated that in producing a test device of the invention all such carrier matrix concepts can be employed, as can others. The matrix can also comprise a system wherein the composition ingredients are homogeneously combined in a fluid or semifluid state, which later hardens or sets, thereby incorporating the ingredients. Other matrix formats are contemplated, including the use of microporous membranes or polymer film matrices. Microporous membranes are available as preformed membranes or can be prepared by such techniques as phase inversion. Suitable polymer films can be produced with commercially available latex formulations based on latex polymer suspensions such as that formed from a 60:40 copolymer of styrene and butadiene. Other natural or synthetic polymers or mixtures thereof can also be used. Examples of such film formulations can be found in US—A—3,630,957 and 4,312,834.

The presently preferred method of preparation is impregnating a bibulous carrier matrix, for example filter paper, with an aqueous solution of the composition and drying, followed by affixing the dried impregnated matrix to a support member. The impregnating solution is prepared so that it exhibits the desired initial pH. When a whole blood sample is to be tested, the dried impregnated carrier matrix can be coated to allow excess sample to be washed or wiped off. Drying can be accomplished by any means which will not deleteriously affect the incorporated composition, usually by means of an air oven. Incorporation can be accomplished by any method such as coating, dipping, spreading, spraying or printing which allows the carrier matrix to be incorporated with the assay composition. The dried carrier matrix can thereafter be cut and mounted on one end of a support member, for example, a rigid or semirigid polystyrene film strip. The dihydroxide component and/or buffer are in such a form as to be capable of providing an initial pH above 6.5 at the surface of the incorporated carrier when the carrier is wetted. The pH of the wetted incorporated carrier can be measured with surface electrodes. The term "incorporated carrier" refers to a carrier matrix incorporated with the test composition and dried. When a transparent film strip is used, instrumental reading of a reacted device can be accomplished from either side of the strip. Mounting of the paper on the strip can be accomplished through use of a double-faced adhesive tape, such as that commercially available from the 3M Co., St. Paul, MN., under the trademark DOUBLE STICK®.

When a paper carrier matrix is used with a boric acid buffer, it can be advantageous to treat the paper with an aqueous solution of a second borate buffer such as that prepared from phenylboronic acid at an initial pH above 6.5, prior to incorporation of the test composition containing the borate buffer. It is speculated that such a pretreatment prevents possible interaction of the paper with the borate buffer in the test composition.

Concentration ranges for components in the reagent solution used to prepare a solid state test device are as follows:

| | Working | Preferred |
|---|---|---|
| Borate buffer | 0.1—0.9M | 0.1—0.4M |
| pH indicator | 0.025—0.2% | 0.04—0.15% |

These concentration ranges and relative concentrations of components are viable whether the solution is an aqueous impregnating solution or a polymer suspension. A preferred reagent solution contains from 0.10 to 0.30 M borate buffer titrated to an initial pH of about 8.5 to about 9.5 and 0.05% to 0.10% of an indicator such as m-cresol purple. In a preferred embodiment a paper matrix is impregnated with an aqueous solution containing from 0.1 to 0.3M of borate buffer titrated to an initial pH above 6.5 prior to incorporation with the test composition. A preferred borate buffer for pretreatment is prepared from phenylboronic acid.

The test device is advantageously used by momentarily dipping it in a test sample or by otherwise introducing a test sample onto the carrier matrix, whereby a detectable colorimetric change results when glucose is present. Contact with the test sample can also be made by pipette, swab or spatula. Although dipping is a highly satisfactory method of contact when urine is used, a serum sample will normally require pipetting.

Semiquantitative glucose concentrations can be determined visually by comparison with an appropriate color chart or measurements can be made instrumentally by reflectance from either side of the device if a transparent support member is used.

Abbreviations

The following abbreviations are used in the examples.

| | |
|---|---|
| mg | milligram |
| ml | milliliter |
| dl | deciliter |
| M | molar |
| % | percent given in weight per 100 ml solution |
| PVP-K90 | poly(vinylpyrrolidone) average molecular weight 360,000 from GAF Corp., New York, N.Y. |
| m-cresol purple | meta-cresolsulfonephthalein |
| cresol red | o-cresolsulfonephthalein |
| neutral red | 2-methyl-3-amino-6-dimethyl aminophenazine |
| thymol blue | thymolsulfonepthalein |
| phenylphthalein | 3,3-bis(p-hydroxyphenyl)-phthalide |
| phenol red | phenolsulfonephthalein |
| bromothymol blue | dibromothymolsulfonephthalein |
| Klucel® LF | hydroxypropyl cellulose |
| polyethylene glycol 4000 | polyethylene glycol, molecular weight 4000 |

Examples

1. Phenylboronic acid buffer pretreatment

Whatman 54 filter paper was immersed in an aqueous solution containing 0.2 M phenylboronic acid buffer, initial pH 9.05. The impregnated paper was then dried for 15 minutes at 60°C in an air oven. The dried paper was immersed in an aqueous solution containing 0.25 M borate buffer, initial pH 9.05, and 0.08% m-cresol purple (sodium salt). A stock solution of 1% m-cresol purple in ethanol was used to prepare the impregnating solution. The borate buffer was prepared by dissolving boric acid in water, adjusting the pH to 9.05 with potassium hydroxide and diluting to the desired volume. The doubly impregnated paper was again dried for 15 minutes at 60°C in an air oven.

A piece of the doubly dried paper was affixed to a polystyrene support member for convenient handling. The test devices were tested by dipping in aqueous test samples containing from 1000 mg/dl to 8,000 mg/dl (i.e., 1 g/dl to 8 g/dl) glucose. Data displayed in the drawing indicates good linear correlation between glucose concentration (g/dl) and the strip reading.

2. No pretreatment

Whatman 54 filter paper was immersed in a solution containing:

| | |
|---|---|
| boric acid buffer (1M, initial pH 9.5) | 2.0 ml |
| PVP K90 (15%) | 1.0 ml |
| Phenol Red (1M) | 0.2 ml |
| Water | 10.0 ml |

The impregnated paper was dried for 15 minutes at 60°C in an air oven and a piece of the dried paper was affixed to support members made of polystyrene. The finished test device provides good visual resolution between 1,000, 2,000, 3,000 and 5,000 mg/dl glucose. The color changes from red for negative (less than 1,000 mg/dl glucose) to yellow (5,000 mg/dl glucose).

3. Double indicator system

A particularly preferred test device for the determination of high range glucose (i.e. concentrations of at least 1,000 milligrams per deciliter) in a urine test sample is prepared as follows:

Solution 1 (10% acetone in water)

| | |
|---|---|
| phenylboronic acid buffer (initial pH 9.0) | 0.23M |
| dodecylbenzenesulfonic acid (sodium salt) | 0.04% |

Solution 2

| | |
|---|---|
| boric acid buffer (initial pH 9.0) | 0.30M |
| PVP-K 60 | 1.2% |
| polyethylene glycol 4000 | 0.8% |
| Klucel® LF (in ethanol) | 0.4% |
| Tween 21® (sorbimacrocol laurate) | 0.04% |
| Cresol Red | 0.03% |
| Bromothymol Blue, (in ethanol) | 0.003% |

Solution 2 is made up in 10% acetone. The final solution contains 10% acetone and 10% ethanol.

Filter paper, such as Whatman 54 or E & D 204, is pretreated by dipping into solution 1 and drying. The dried pretreated paper is dipped into solution 2 and dried. The dual indicator system facilitates the semiquantitative differentiation of glucose concentrations between 1,000 mg/dl and 10,000 mg/dl as the difference in colorimetric response between different concentration levels is greater. This is particularly desirable for a visually read test device.

**Claims**

1. A test composition for the semiquantitative determination of at least 1,000 milligrams per deciliter glucose in an aqueous test sample comprising (a) a borate buffer capable of moderating pH change in a pH range of from pH 6.5 to pH 12; and (b) a pH indicator capable of providing a detectable colorimetric response in a pH range from pH 6.5 to pH 12.

2. A test device for the semiquantitative determination of at least 1,000 milligram per deciliter glucose in an aqueous test sample, comprising: a carrier matrix and a test composition incorporated therewith, the test composition including (a) a borate buffer capable of moderating pH change in a pH range of from pH 6.5 to pH 12; and (b) a pH indicator capable of providing a detectable colorimetric response in a pH rage of from pH 6.5 to pH 12.

**Patentansprüche**

1. Testzusammensetzung für die semiquantitative Bestimmung von wenigstens 1,000 mg/dl Gluclose in einer wäßrigen Testprobe, enthaltend (a) einen Boratpuffer, der in der Lage ist, eine pH-Veränderung in einem pH-Bereich von pH 6,5 bis pH 12 abzuschwächen und (b) einen pH-Indikator, der in der Lage ist, eine nachweisbare kolorimetrische Ansprechung in einem pH-Bereich zwischen pH 6,5 und 12 zu ergeben.

2. Testvorrichtung für die semiquantitative Bestimmung von wenigstens 1,000 mg/dl Glucose in einer wäßrigen Testprobe, umfassend: eine Trägermatrix und eine Testzusammensetzung, welche in diese eingebracht wurde, wobei die Testzusammensetzung (a) einem Boratpuffer, der in der Lage ist, eine pH-Veränderung in einem pH-Bereich zwischen 6,5 und 12 abzuschwächen und (b) einen pH-Indikator, der in der Lage ist, eine nachweisbare kolorimetrische Ansprechung in einem pH-Bereich zwischen 6,5 und 12 zu ergeben.

**Revendications**

1. Composition pour test pour la détermination semiquantitative d'au moins 1,000 milligrammes par décilitre de glucose dans un échantillon aqueux pour test comprenant (a) un tampon borate capable de modérer la variation du pH dans une gamme de pH de pH 6.5 à pH 12; et (b) un indicateur du pH capable de fournir une reponse colorimetrique détectable dans une gamme de pH de pH 6.5 à pH 12.

2. Dispositif pour test pour la détermination semiquantitative d'au moins 1,000 milligrammes par decilitre de glucose dans un échantillon aqueux pour test, comprenant: une matrice porteuse et une composition pour test qui y est incorporée, la composition pour test comprenant (a) un tampon borate capable de modérer la variation du pH dans une gamme de pH de pH 6.5 à pH 12; et (b) un indicateur du pH capable de fournir une résponse colorimétrique détectable dans une gamme de pH de pH 6,5 à pH 12.

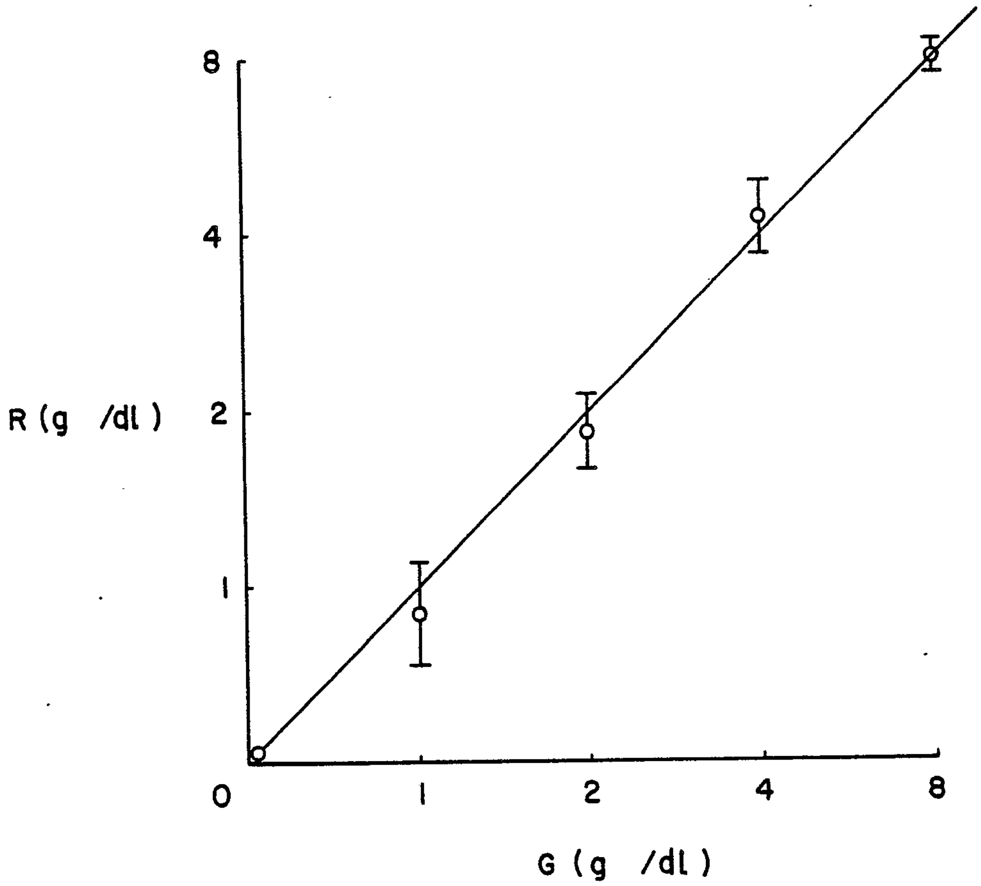
8
4
2
1
R (g /dl)
0
1
2
4
8
G (g /dl)